# EUROPEAN PATENT SPECIFICATION

(11) **EP 3 207 880 B1**
(45) Date of publication and mention of the grant of the patent: **24.07.2019**
(21) Application number: 16193077.1
(22) Date of filing: 17.06.2004
(51) Int. Cl.: A61B 10/06, A61B 17/29, A61B 17/28, A61B 17/00, A61B 17/32

(54) **ENDOSCOPIC INSTRUMENT**
ENDOSKOPISCHES INSTRUMENT
INSTRUMENT ENDOSCOPIQUE

(30) Priority: 18.06.2003 US 479145 P; 17.02.2004 US 778226; 14.05.2004 US 845108
(43) Date of publication of application: 23.08.2017
(62) Divisional of application: 10012252.2
(73) Proprietor: Boston Scientific Limited, Hamilton HM11 (BM)
(72) Inventor: ROTHBERG, Eliott, Coral Spring, FL 33076 (US); GIL DE MONTES, William, Pembroke Pines, FL 33029 (US); ZARDESKAS, James M., Pascoag, RI 02859 (US); FREED, David I., Westborough, MA 01581 (US); MAGILL, Michael J., Northborough, MA 01532 (US); SHARMA, Satish, Randolph, MA 02368 (US); GINGRICH, Jon, Lancaster, PA 17601 (US); BOARINI, Edward, Holliston, MA 01746 (US)
(74) Representative: Vossius & Partner Patentanwälte Rechtsanwälte mbB

(56) References cited:
- JP-A- 2002 153 475
- US-A- 2 796 065
- US-A- 3 101 715
- US-A- 5 059 214
- US-A- 5 553 624

## Description

### Field of the Invention

Embodiments of the invention include a medical device with one or more of a variety of features. More particularly, embodiments of the invention relate to endoscopic devices that include one or more features that improve the use of the device. Examples of such features include chamfered edges and corners on, for example, the end effectors, a surface with a controlled finish also on, for example, the end effectors, a jaw with teeth and/or a tang having various configurations, a handle having soft-grip features, and/or an elongate member with varied rigidity. Other examples of such features include a folded portion on, for example, the end effectors and/or a snap-fit clevis assembly.

### Background of the Invention

Various medical instruments may be used in connection with an endoscope for performing a number of operations at a site deep within a patient's body cavity. One such instrument, a biopsy forceps device, samples tissue from a body cavity with minimal intervention and discomfort to patients. Typically, a biopsy forceps device, like other endoscopic instruments, has a long flexible tubular member for insertion into a lumen of an endoscope. The tubular member is sufficiently long and flexible to follow a long, winding path of the body cavity. An end effector assembly, such as a biopsy forceps assembly, is attached at a distal end of the tubular member, and a handle is attached at a proximal end of the tubular member. The handle may have an elongate portion and a spool portion disposed around the elongate portion. The spool portion may be configured to move longitudinally relative to the elongate portion. An elongate mechanism, such as one or more pull wires, extends through the tubular member to connect the end effector assembly and a portion of the handle, such as the spool portion. Longitudinal movement of the spool portion relative to the elongate portion of the handle causes the elongate mechanism to move longitudinally in the tubular member, which in turn causes the actuation of the end effector assembly.

In methods of using the biopsy forceps device, an endoscope is placed in a patient's body cavity adjacent a tissue site from which the acquisition of a tissue sample is desired. The biopsy forceps device is then advanced to the tissue site via a working channel of the endoscope. Once the biopsy forceps device is next to the portion of the tissue from which the acquisition of a tissue sample is desired, the spool portion is moved relative to the elongate portion so as to move pull wires. The movement of the pull wires causes the jaws of the biopsy forceps assembly to open. The open jaws are then advanced to the tissue site, and the spool portion is again moved relative to the elongate portion so as to move the pull wires such that the jaws close. The closing of the jaws causes a tissue sample to be captured in the end effector assembly. The biopsy forceps device is then removed from the body cavity via the working channel of the endoscope.

US 3 101 715 A relates to a non-crushing clamp particularly useful for gripping body tissues during surgery.

US 2 796 065 A relates to forceps and clamping devices used in surgery to grip, clamp, restrict and coarctate tubular body vessels and tissues.

JP 2002 153475 A relates to a forceps for an endoscope provided with a pair of openable and closable forceps pieces.

US 5 059 214 A relates to surgical forceps for grasphing and holding tissues or organs during various manipulations in the operative wound in the course of surgical procedures.

US 5 553 624, representing the closest prior art, relates to an endoscopic biopsy forceps instrument having a pair of opposing distal jaws, each jaw provided with a toothed cup.

### SUMMARY OF THE INVENTION

The present invention is defined by the independent claim.

An example of the disclosure includes a medical device including a handle, an end effector assembly, and a member connecting the handle to the end effector assembly. The end effector assembly includes an end effector having non-sharp edges and corners.

Another example of the disclosure includes a medical device including a handle, an end effector assembly, and a member connecting the handle to the end effector assembly. Portions of the end effector assembly have a roughened surface.

An embodiment of the invention includes a medical device including a handle, an end effector assembly, and a member connecting the handle to the end effector assembly. The end effector assembly includes opposing jaw portions each including a plurality of teeth. Each of the teeth includes a crest, a root, and an intermediate portion between the crest and the root. The intermediate portions of opposing jaw portions are configured to contact each other when the opposing jaw portions are brought together and the root has a recessed portion configured to accommodate a sharp, pointed tip of the crest.

Still another example of the disclosure includes a medical device including a handle, an end effector assembly, and a member connecting the handle to the end effector assembly. The end effector assembly includes at least one end effector having a tang defining a mounting hole configured to receive one of a wire and an axle and the tang includes a portion disposed around the mounting hole that has a thickness greater than a thickness of other portions of the tang.

A further example of the disclosure includes a medical device including a handle, an end effector assembly, and a member connecting the handle to the end effector assembly. The end effector assembly includes at least one biopsy jaw having a tissue receiving portion that defines at least one hole configured so as to substantially prevent contact between an edge of the hole and a tube-like member in which the end effector assembly is configured to extend through.

A yet further example of the disclosure includes a medical device including a soft-grip handle, an end effector assembly, and a member connecting the handle to the end effector assembly.

A still further example of the disclosure includes a medical device including a handle, an end effector assembly, and an elongate, flexible member connecting the handle to the end effector assembly. A proximal portion of a distal third of the elongate member is more flexible than adjacent portions of the elongate member.

Another example of the disclosure includes a medical device including a handle, an end effector assembly, and an elongate, flexible member connecting the handle to the end effector assembly. The end effector assembly includes a pair of opposing biopsy jaws each having a tissue receiving portion having a roughened surface and defining a hole, the hole configured so as to substantially prevent contact between an edge of the hole and a tube-like member in which the end effector assembly is configured to extend through. Each biopsy jaw further includes a tang defining a mounting hole configured to receive one of a wire and an axle, the tang including a portion disposed around the mounting hole that has a thickness greater than a thickness of other portions of the tang. Each biopsy jaw further includes a plurality of teeth, each of the teeth including a crest, a root, and an intermediate portion between the crest and the root. The intermediate portions of opposing biopsy jaws are configured to contact each other when the biopsy jaws are brought together, and the root has a recessed portion configured to accommodate a sharp, pointed tip of the crest.

Various examples may have any or all of the following features: wherein the end effector defines a hole having a non-sharp edge. The end effector may include a jaw extending from an arm, and wherein all edges of the jaw other than a cutting edge of the jaw are non-sharp. The non-sharp edges and corners may be beveled. Portions of the end effector assembly may have a rougher surface than other portions of the end effector assembly. The end effector assembly may include a biopsy forceps jaw having a roughened surface. The roughened surface of the biopsy forceps jaws may be an outer surface of the biopsy forceps jaw. The roughened surface of the biopsy forceps jaws may be an inner surface of the biopsy forceps jaw. The roughened surface may be formed by one of grit blasting, media tumbling, plating, sputtering, photo-etching, acid-etching, and plasma coating. The root may be at least a partial, substantially circular cutout. A center of the cutout may be displaced vertically relative to adjacent intermediate portions. A center of the cutout may be displaced horizontally relative to a center of adjacent intermediate portions. The root may be a U-shaped groove. A center of the U-shaped groove may be displaced vertically relative to adjacent intermediate portions. A center of the U-shaped groove may be displaced horizontally relative to a center of adjacent intermediate portions. A gap may be between the tip and the root of opposing teeth when the opposing jaw portions are brought together. A wire having a first wire portion may be substantially contacting one side of the tang and a second wire portion substantially contacting another side of the tang. The at least one end effector may include two end effectors. The wire may be bent on both sides of the mounting hole. A section of the tang defining a through hole may be folded so that the through hole is substantially aligned with the mounting hole. The at least one end effector may define a second mounting hole configured to receive the other of the wire and the axle, and wherein the tang includes a second portion around the second mounting hole that has a thickness greater than the thickness of other portions of the tang. The hole may be disposed off a centerline of the biopsy jaw. The at least one biopsy jaw may include two biopsy jaws. The at least one hole may include a plurality of holes. The handle may have a ring portion connected to an elongate portion, and a spool portion disposed around the elongate portion, and wherein the ring portion and the spool portion have a soft-grip configuration. The handle may have a plurality of finger rings, and wherein the finger rings have a soft-grip configuration. The soft-grip handle may include a low durometer material. The soft-grip handle may include at least one of santoprene and urethane.

A further example of the disclosure includes an end effector assembly for a medical instrument. The end effector assembly includes an end effector having a tang defining a pivot hole. An edge of the tang proximal to the pivot hole extends within an outer periphery of the tang.

Still another example of the disclosure includes a medical device. The medical device includes a handle, an end effector assembly, and a member connecting the handle to the end effector assembly. The end effector assembly includes an end effector having a tang defining a pivot hole. An edge of the tang proximal to the pivot hole extends within an outer periphery of the tang.

Various examples may have any or all of the following features: the tang may be configured to substantially prevent contact between the edge and a channel in which the end effector assembly is configured to extend through, as the end effector pivots about the pivot hole; a section of the tang at the outer periphery adjacent the edge may have a smooth surface; a first tang portion extending from the outer periphery to the edge may form less than a 90 degree angle to a second tang portion extending from the outer periphery and defining the pivot hole; the first tang portion and the second tang portion may form an approximately zero degree angle; the first tang portion and the second tang portion may be substantially parallel; a section of the tang between the outer periphery adjacent the edge and the edge may be curved; the edge may be substantially sharp.

A still further example of the disclosure includes a clevis assembly for a medical instrument. The clevis assembly includes a clevis having a base and a first arm and a second arm extending from the base and an axle extending between the first arm and the second arm. The axle defines a groove in which a portion of the first arm is disposed.

Yet another example of the disclosure includes a clevis assembly for a medical instrument. The clevis assembly includes a clevis having a base and a first arm and a second arm extending from the base and an axle extending between the first arm and the second arm. A portion of the first arm is configured to deflect from an original configuration and return to the original configuration as the axle is placed through the first arm.

A yet further example of the disclosure includes a medical instrument. The medical instrument includes a handle portion, an end effector assembly, and an elongate member connecting the handle portion to the end effector assembly. The end effector assembly includes a clevis having a base and a first arm and a second arm extending from the base and an axle extending between the first arm and the second arm. The axle defines a groove in which a portion of the first arm is disposed.

Another example of the disclosure includes a medical instrument. The medical instrument includes a handle portion, an end effector assembly, and an elongate member connecting the handle portion to the end effector assembly. The end effector assembly includes a clevis having a base and a first arm and a second arm extending from the base and an axle extending between the first arm and the second arm. A portion of the first arm is configured to deflect from an original configuration and return to the original configuration as the axle is placed through the first arm.

Various examples may have any or all of the following features: the portion may be configured to deflect from an original configuration as the axle is placed through the first arm; the portion may be configured to substantially return to the original configuration for disposition in the groove; the portion may include a plurality of protrusions defining a hole in the first arm; the protrusions may deflect; the second arm may define a hole, and a portion of the axle at an end opposite the groove may be configured to prevent passage of the portion of the axle through the hole; an end of the axle may have a larger circumference than a central portion of the axle; and the axle may include end portions having cross-sectional sizes larger than a hole defined by the portion of the first arm.

A further example of the disclosure includes a method of manufacturing an end effector assembly of a medical instrument. The method includes providing a clevis having a base and a first arm and a second arm extending from the base, providing an axle, placing an axle through the second arm, placing the axle through the first arm so as to deflect a portion of the first arm, and returning the portion of the first arm to its original configuration.

Various examples may have any or all of the following features: the portion of the first arm in a groove on the axle; providing an end effector; placing the axle through a portion of the end effector.

Additional objects and advantages will be set forth in part in the description which follows, and in part will be obvious from the description, or may be learned by practice. The objects and advantages of the invention will be realized and attained by means of the elements and combinations particularly pointed out in the appended claims.

The foregoing general description and the following detailed - description are exemplary and explanatory only and are not restrictive of the invention, as claimed.

### BRIEF DESCRIPTION OF THE DRAWINGS

The accompanying drawings, which are incorporated in and constitute a part of this specification, illustrate examples and together with the description, serve to explain the principles of the invention.
Fig. 1 is a perspective view of an endoscopic instrument suitable for use in connection with embodiments of the present invention.
Fig. 2 is a perspective view of a jaw portion of an endoscopic instrument.
Fig. 3 is a perspective view of a jaw portion of an endoscopic instrument according to example of the disclosure.
Fig. 4 is a schematic view of an endoscopic instrument with an elongate member of variable flexibility according to an example of the disclosure.
Fig. 5 is a perspective view of a jaw assembly of an endoscopic instrument according to an example of the disclosure.
Fig. 6 is a perspective view of a jaw assembly of an endoscopic instrument according to an example of the disclosure.
Fig. 7 is a view of a jaw portion of the jaw assembly of Fig. 6.
Fig. 8A is a side view of mated jaw portions of an endoscopic instrument.
Fig. 8B is a side view of mated jaw portions of an endoscopic instrument.
Fig. 9 is a side view of a jaw portion of the jaw assembly of Fig. 6.
Fig. 10 is a side view of the mated jaw portions of Fig. 9.
Fig. 11 is a side view of a jaw portion of an endoscopic instrument according to another embodiment of the present invention.
Fig. 12 is a side view of a jaw portion of an endoscopic instrument according to yet another embodiment of the present invention.
Fig. 13 is a top view of a tang portion and control wire of an endoscopic instrument.
Fig. 14 is a top view of a tang portion and control wire of an endoscopic instrument according to an example of the disclosure.
Fig. 15A is a side view of a jaw with a tang portion, having an unfolded additional section, of an endoscopic instrument according to another example of the disclosure.
Fig. 15B is a perspective view of the jaw with the tang portion of Fig. 15A, with the additional section folded.
Fig. 16 is a side view of a handle of an endoscopic instrument according to an example of the disclosure.
Fig. 17 is a side view of a handle of an endoscopic instrument according to another example of the disclosure.
Fig. 18A is a side view of a tang portion of a jaw according to a further example of the disclosure.
Fig. 18B is a cross-sectional view of the tang portion of Fig. 18A along line 18B-18B.
Fig. 18C is a cross-sectional view of a tang portion of a jaw according a still further example of the disclosure.
Fig. 18D is a cross-sectional view of a tang portion of a jaw according a yet further example of the disclosure.
Fig. 19A is a perspective view of a clevis assembly according to yet another example of the disclosure.
Fig. 19B is a side view of an axle of the clevis assembly of Fig. 19A.
Fig. 19C is a partial side view of a portion of the clevis assembly of Fig. 19A.
Fig. 19D is a schematic view of the clevis assembly of Fig. 19A.
Fig. 19E is a schematic view of the clevis assembly of Fig. 19A, with the axle being inserted into the clevis.
Fig. 20A is a side view of a clevis according to still another example of the disclosure.
Fig. 20B is a schematic view of an axle in the clevis of Fig. 20A.
Fig. 20C is a schematic view of the axle and clevis of Fig. 20A.

### DESCRIPTION OF THE EMBODIMENTS

Reference will now be made in detail to the present examples illustrated in the accompanying drawings. Wherever possible, the same reference numbers will be used throughout the drawings to refer to the same or like parts.

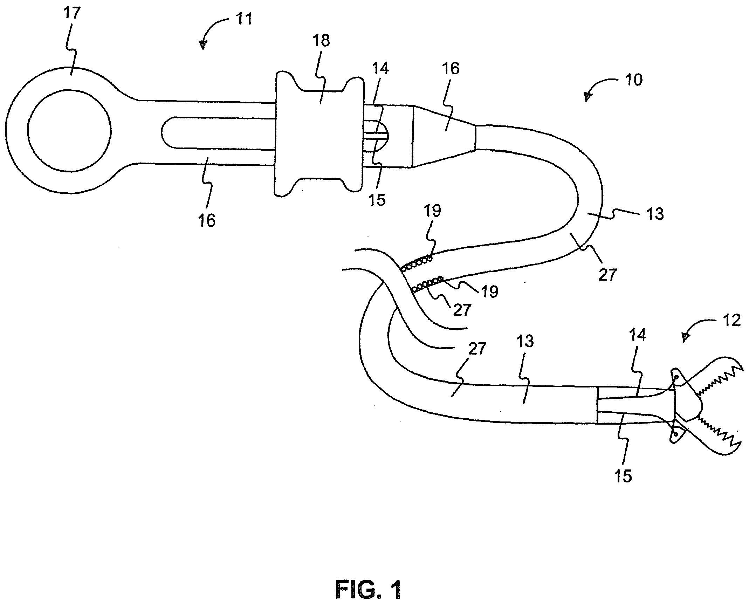

An exemplary embodiment of a medical device is depicted in Fig. 1. The medical device is an endoscopic instrument 10 that includes a handle portion 11 and an end effector assembly 12 connected to each other by a flexible elongate member 13. Control wires 14, 15 extend between the handle portion 11 and the end effector assembly 12 via a lumen in the flexible elongate member 13. The handle portion 11 includes an elongate portion 16 connected at its proximal end to a ring portion 17 and a spool portion 18 slidably disposed around the elongate portion 16. The elongate member 13 may having a coiled portion 19 (partially shown in Fig. 1) covered by an outer jacket or a sheath 27. However, the elongate member 13 may not have a coiled portion 19, and instead may include a single layer tubular member. The end effector assembly 12 may be any type of assembly, for example, a biopsy forceps jaw as depicted in Fig. 1. The control wires 14, 15 may be connected at their distal ends to opposing portions of the end effector assembly 12, and at their proximal ends to spool portion 18. Longitudinal movement of the spool portion 18 relative to the elongate portion 16 causes the actuation of the end effector assembly 12 via the control wires 14, 15. Portions of the control wires 14, 15 disposed in the handle 16 may be contained within a tube also disposed in the handle 16. The tube may provide the compressive strength that may be needed to actuate the end effector assembly 12.

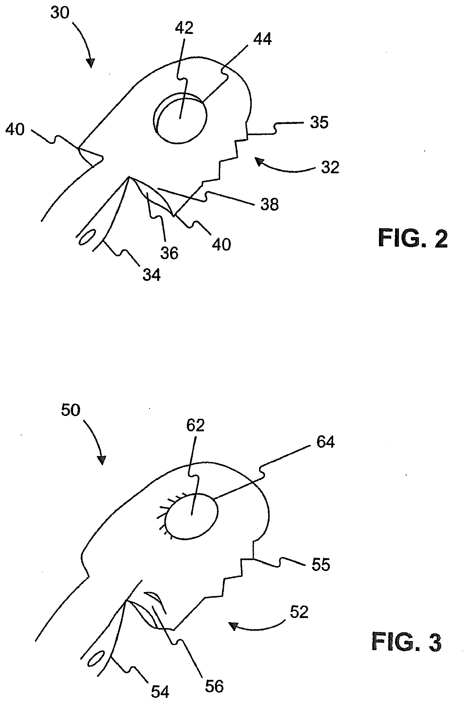

A current biopsy forceps jaw 30, such as that shown in Fig. 2, includes a jaw 32 extending from an arm 34. Jaw 32 includes a sharp edge or teeth 35 at its cutting edge. Teeth 35 may mate with another biopsy forceps jaw, of like or similar construction, of an endoscopic forceps instrument to obtain a biopsy sample. Jaw 32 also includes flat surfaces on various parts of jaw 32. For example, the back or proximal-most surface 36 of jaw 32 and certain surfaces intersecting with surface 36 may be flat. The intersection of those surfaces will result in sharp corners and edges, such as edges 38 and corners 40. Jaw 32 also defines a fenestration hole 42 that may include a sharp edge 44. Many current biopsy forceps jaws have such a construction because they are cast from a molded plastic pattern. Certain efficiencies in the manufacture of injection molds lead to flat, intersecting planes and sharp edges and corners of the resultant jaws. These sharp edges and corners, however, may get caught within an endoscope working channel upon entry or exit of a biopsy forceps device through that channel or at the distal end of the endoscope upon re-entry of the forceps after use.

Examples include a medical device or portions of the medical device with chamfered corners and/or edges. Fig. 3 shows a biopsy forceps jaw 50 according to an example. The biopsy forceps jaw 50 includes a jaw 52 extending from an arm 54. Like jaw 32 of Fig. 2, jaw 52 includes a sharp edge or teeth 55 at a cutting edge. Unlike jaw 32, however, certain surfaces of jaw 52 are not substantially flat and, instead, are rounded at least near the edges of those surfaces. The corners and edges of various intersecting surfaces are therefore chamfered, beveled, rounded, and/or radiused off and not sharp. For example, the back or proximal-most surface 56 of jaw 52 and certain surfaces intersecting with surface 56 are rounded at least near the edges of those surfaces so that there are no, or fewer, sharp edges or corners associated with jaw 32 (other than the sharp cutting edge having teeth). Jaw 52 also defines a fenestration hole 62 that may include an edge 64 that is rounded, chamfered, beveled, and/or radiused, off so that there is not a sharp edge. The resulting jaw will have no, or fewer, sharp edges or corners to catch within an endoscope working channel upon entry or exit of a biopsy forceps device through that channel or at the distal end of the endoscope upon re-entry of the forceps after use. Less interference with at least the distal section of the endoscope results.

Providing a medical device, or portions thereof, with non-sharp edges and corners may apply to other types of end effectors or other parts of endoscopic or non-endoscopic instruments, including, but not limited to graspers, scissors, forceps, or other laproscopic, endoscopic, or other devices. For example, the medical device may have a sharp cutting edge that is a radial edge (i.e., a straight cutting edge with no teeth). Other edges, corners, and surface intersections, aside from those mentioned above, may be rounded, chamfered, beveled, and/or radiused off as desired to minimize the effects associated with sharp regions as the device is being used. For example, other portions of the end effector assembly, including tang portions, clevis portions, and/or axle portions may include rounded, chamfered, beveled, and/or radiused off edges and corners.

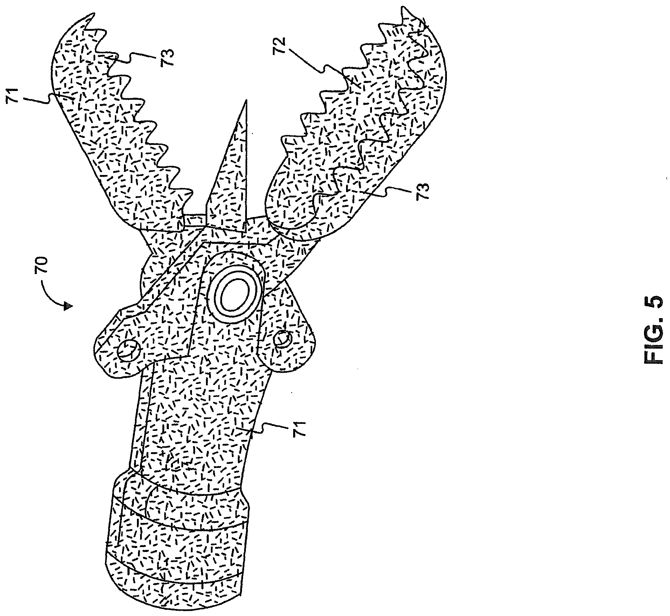

Examples include a medical device or portions of the medical device having a controlled surface finish, including a roughened surface finish. Fig. 5 shows the inner surface 72 and outer surface 71 of a biopsy forceps jaw assembly 70 having a rough surface finish. While Fig. 5 shows a biopsy forceps jaw assembly 70 having all parts with a roughened surface, less than all of the parts of the jaw assembly 70 may include a roughened or textured surface. For example, to attain many of the advantaged described herein, it may be desirable for only the jaws 73, or portions of the jaws 73 such as the outer surface 71, to have a roughened or otherwise textured surface.

Tissues are less prone to sticking to surfaces of jaws having a rough finish than surfaces of jaws having a smooth finish. For example, tissue samples cut with the roughened jaws 73 may be less prone to sticking to the surfaces 71, 72 of the jaws 73. By lessening the smoothness of the inner surface 72 of the jaws 73, the tissue sample may be more easily removed from the jaws 73, for example, when the tissue sample is discharged into an external container.

One potential advantage of having a controlled roughness on the surface of the jaws is that by reducing the amount of sticking, surface contact, and/or seal between the tissue samples and the biopsy jaws, the amount of time spent in a biopsy tissue acquisition procedure is reduced. For example, the amount of time spent trying to release the surface contact between the tissue samples and the surfaces of the jaws, during multiple sample acquisition and/or removing the samples from the jaws into an external specimen container, is reduced. This may permit faster turnaround when a single bite biopsy forceps assembly needs to be removed from an endoscope, the tissue sample retrieved from the jaw, and the assembly reinserted into the endoscope to obtain a subsequent sample.

Another potential advantage for having a rough finish on the surface of the endoscopic instrument is that it reduces surface contact between jaws and/or prevents surfaces of the jaws from sealing and/or sticking to each other. Smooth surfaces may sometimes stick together and form a seal, particularly if a fluid is placed between the surfaces. Having a rough finish on the surface of the jaws reduces the force with which that particular surface of the jaws will stick to either each other or another surface. For example, the surfaces of the teeth of opposing jaws may be less prone to sticking to each other when brought together.

Yet another potential advantage for controlling the surface finish of an endoscopic instrument is that it may provide a more consistent feel and/or performance to the user. For example, the entire endoscopic instrument may have a particular finish, or portions of the endoscopic instrument, such as the end effectors, may have different finishes.

A further potential advantage for controlling the surface finish of an endoscopic instrument is that, for example, when an optimum level of roughness is provided to the surface of the jaw assembly, tissue is more readily grasped and retained in the jaws, for example, so that multiple samples may be collected with a single bite forceps. The controlled surface texture may allow a user to obtain subsequent tissue samples with the prior sample(s) remaining within the jaws. A particular texture of the jaws may allow the tissue sample to be retained within the open jaws while the user acquires a second sample.

A still further potential advantage for controlling the surface finish of an endoscopic instrument is that, for example, when an optimum level of roughness is provided to the surface of the jaw assembly, the roughened surface may assist in both retaining and removing the sample. Such assistance may be dependent on the presence or absence of an external force. For example, when there is no external force exerted on the sample, the roughened surface may assist in the retention of the sample. In another example, when an external force is applied to the sample, the roughened surface may assist in the removal of the sample.

The roughness of the surfaces 71, 72 of the jaw assembly 70 may be created and/or adjusted, for example, by controlling the casting of the jaws 73 and/or subsequent processing of the jaw assembly 70. Subsequent processing may including grit blasting, media tumbling, and/or any other suitable surface finishing technique. The surfaces 71, 72 of the jaw assembly 70 could also be plated, sputtered, photo-etched, acid-etched, and/or plasma coated to control the roughness of the surface. The surface or surfaces of the endoscopic instrument may have a roughness in the range of a few hundred microinches, and may be varied, for example, by increments of a few hundred microinches. The relative roughness of the surface or surfaces of the endoscopic instrument may be varied with respect to each other. For example, one surface or portion of a surface may have a relatively rough finish, while another surface or portion of a surface may have a relatively smooth finish.

Providing surface(s) of a medical device, or portions thereof, with a controlled finish, for example a roughened surface, may apply to other types of end effectors or other parts of endoscopic or non-endoscopic instruments, including, but not limited to graspers, scissors, forceps, or other laproscopic, endoscopic, or other devices. Furthermore, other portions of the end effector assembly, including tang portions, clevis portions, and/or axle portions may include surfaces with a controlled finish, for example, a roughened surface. Additionally, only specific portions of parts of the end effector assembly may have a controlled finish. For example, only the inner surfaces of a the jaws of an end effector assembly may have a roughened surface.

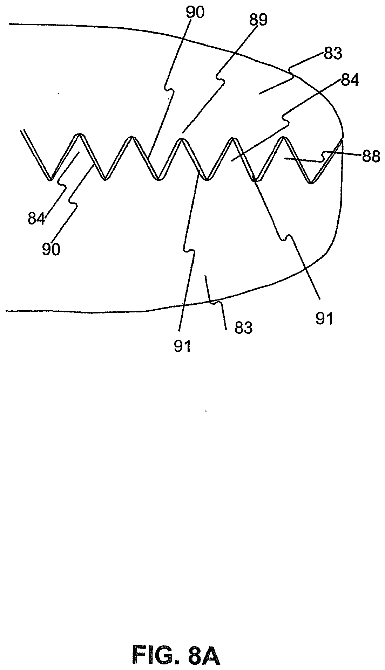

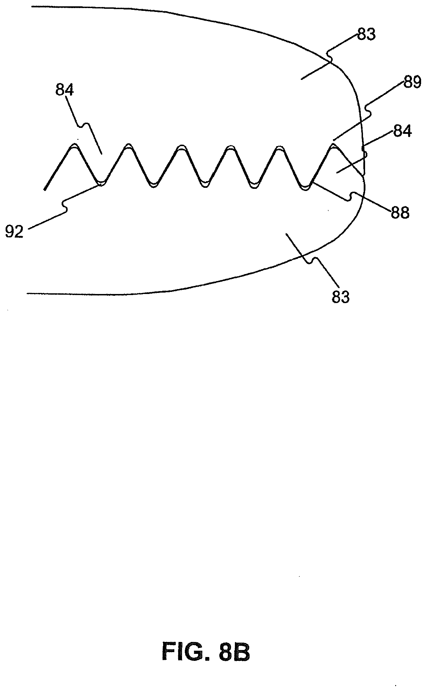

Views of mated jaw portions 83 of endoscopic instruments are shown in Figs. 8A and 8B. Each jaw portion 83 has teeth 84, with each tooth 84 having a crest portion 88. A root portion 89 is disposed between each set of adjoining teeth 84. Substantially diagonal portions 90 of the teeth 84 are disposed between the crest 88 and the root 89 to form the tooth.

The configuration of the root 89 may limit the configuration of the teeth. For example, in order for opposing teeth 84 to fit together, the substantially diagonal portions 90 of teeth 84 on opposing jaw portions 83 need to meet before the crest 88 contacts the root 89. Otherwise, a gap 91 will form between the substantially diagonal portions 90 of opposing jaw portions 83, as shown in Fig. 8A. The gap 91 may prevent the opposing jaw portions 83 and teeth 84 from performing an effective cutting action. Though Fig. 8A includes jaws 83 having teeth 84 with sharp tips to enhance biting action, it may be difficult to fabricate jaws (such as through stamping) that have matching sharp-cornered roots 89.

In some cases, to ensure the opposing jaws portions 83 fully close, as shown in Fig. 8B, the crest portion 88 may be given a radius (about 0.0127 cm (0.005 inches)) slightly larger than the radius of the root portion 89 (such as about 0.0076 cm (0.003 inches)) A gap 92 is formed between the crest portion 88 of one jaw portion 83 and the root portion 89 of an opposing jaw portion 83. However, this jaw configuration includes teeth with non-sharp tips (i.e. crests) inhibiting optimal cutting performance.

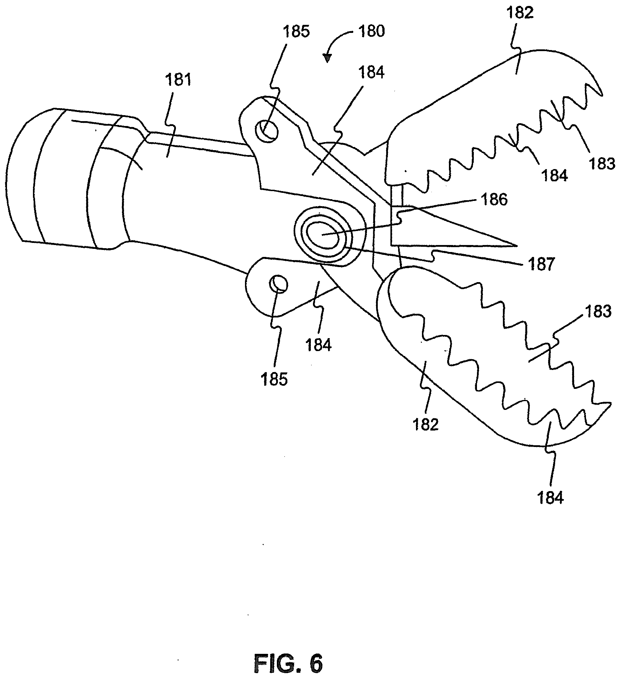

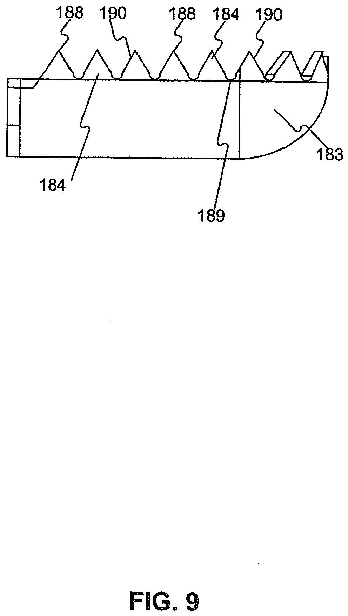

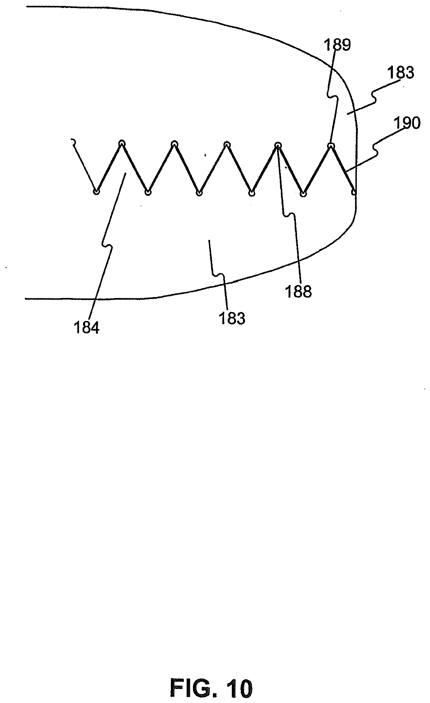

Examples include a medical device having jaws with various tooth and/or teeth configurations that overcome one or more of the drawbacks. A jaw assembly 180 according to an example is depicted in Figs. 6, 7, and 9. The jaw assembly 180 includes a clevis 181 configured to be connected to the end of an elongate member 13. Opposing jaws 182 are rotatably attached to the distal end of the clevis 181. Each jaw 182 has a jaw portion 183 connected to a tang portion 184 with mounting holes 185 on the proximal end of the tang portion 184. The holes 185 may be configured to receive and/or retain a wire 15 or other interface device via the clevis 181. Each tang portion 184 also has an axle hole 186 configured to receive an axle 187 that may be connected to the clevis 181. Each jaw portion 183 has a plurality of teeth 184 configured to mate with the plurality of teeth 184 disposed on an opposing jaw portion 183. Material may be removed from the root 189 of adjoining teeth 184 so that, for example, sharper teeth (i.e., crest portions with smaller or no radii) may be used. As shown in Fig. 9, the root 189 has a circular cutout below the point where the crest 188 of an opposing jaw portion 183 would be captured, regardless of the sharpness of the crest 188 (i.e., the crest 188 may have a substantially zero radius). An example of such a configuration is depicted in Fig. 10. Accordingly, the crest 188 may be as sharp as desired, while still allowing the substantially diagonal portions 190 of opposing jaw portions 183 to come into contact with each other. Methods of sharpening teeth 184 such that the crest 188 has a substantially zero radius are known in the art (e.g., stamping, filing, casting). This jaw portion 183 configuration is advantageous as a sharper crest 188 results in a sharper tooth with an improved bite performance.

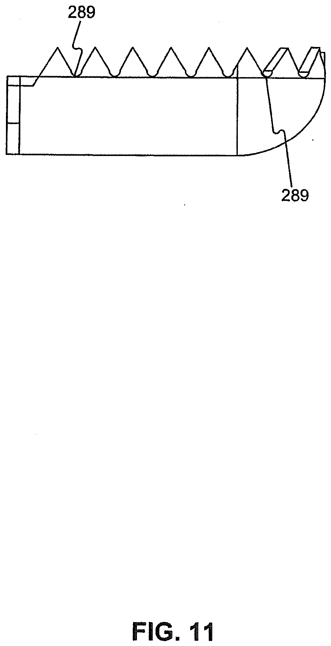

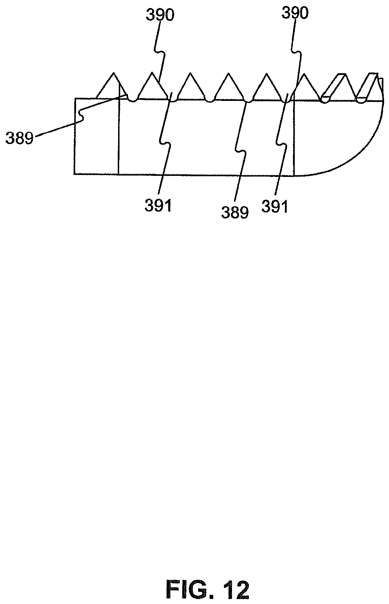

According to the invention, the cutout portions of the root may have any shape or configuration that permits contact between substantially diagonal portions of opposing jaws that include sharp teeth. For example, Fig. 11 shows a root 289 configuration where the cutout is substantially U-shaped. In another example, Fig. 12 shows a root 389 configuration where the circular cutout is shifted vertically. Each root 389 has a center 391 that is disposed below the lower end of the substantially diagonal surfaces 390. In yet another example, the root portion and/or the circular cutout may also be shifted horizontally, so long as the substantially diagonal portions of the opposing jaw portions come into contact with each other without crests contacting the corresponding roots. According to the invention, there is a gap between the tip of the crest and the root, however, according to an example not forming part of the invention, the tip of the crest may also just touch the lowest point of the root.

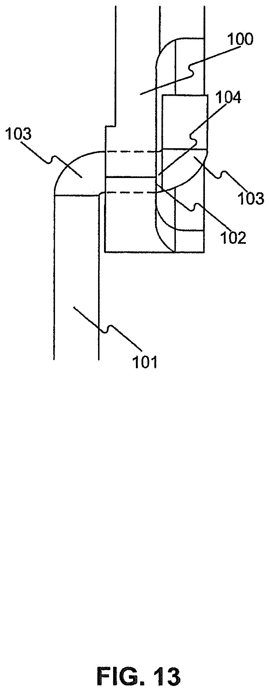

Fig. 13 shows a profile of a tang portion 100 of an end effector assembly for a medical instrument, with a wire 101 disposed in a mounting hole 102 of the tang portion 100. The end portion of the wire 101 has a roughly Z-shaped configuration so as to lodge the wire 101 in the hole 102, allow the wire 101 to rotate with respect to the hole 102, and/or prevent the wire 101 from falling out of the hole 102. The wire end portion has two bends 103 with an interface portion 104 between the bends 103 that contacts the internal surface of the hole 100. The interface portion 104 has substantially the same length as the axial length of the hole 102 and/or the width of the tang 100, for example, to prevent the wire 101 from shifting in the hole 102 and/or falling out of the hole 102. Two methods of forming the roughly Z-shaped configuration (i.e., bends 103) include stamping and/or forging a straight wire 101 into the roughly Z-shaped configuration, however, any method known in the art may be used. If the Z-shape is formed by a stamping or forging operation, the minimum length of the interface portion 104 (i.e., the portion of the wire between the bends) that may be formed is about 0.0381 cm (0.015 inches).

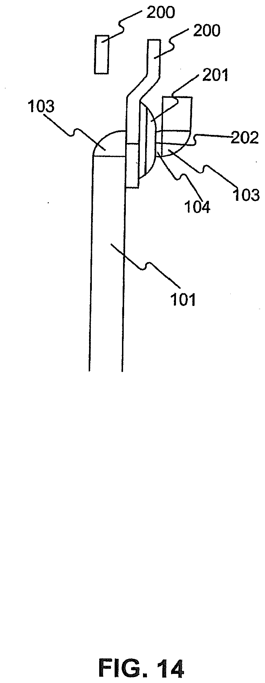

Examples include a medical device having an end effector assembly with various tang configurations. In an example, a substantially narrow tang portion may have a widened portion, for example, by placing a dimple 201 on a tang portion 200 around a mounting hole 202. For example, as shown in Fig. 14, the dimple 201 may extend from the surface of the tang portion 200 and increase the width of the tang portion 200. The dimple 201 may be stamped onto the tang portion 200 so as to increase the width of the tang portion 200. This is advantageous because it allows the tang portion 200 and/or the rest of the jaw assembly to have a smaller thickness while still allowing the jaw assembly to accommodate the end portion of the wire 101 set forth above. Specifically, it allows the thickness of the tang portion 200 without the dimple 201 to be reduced, while still allowing the tang portion 200 and/or the mounting hole 202 to receive and accommodate an end portion of a wire 101 with an interface portion 104 having a length of about 0.0381 cm (0.015 inches). For example, if the width of the tang portion 200 is about 0.0178 cm (0.007 inches), a dimple 201 of about 0.0203 cm (0.008 inches)could be added to the tang portion 200 so as to accommodate an end portion of a wire 101 with an interface portion 104 having a length of about 0.0381 cm (0.015 inches), without the end portion of the wire 101 undesirably shifting in and/or falling out of the mounting hole 202. This is especially advantageous when manufacturing a stamped jaw (with tang) having a thickness of material that is less than the length of the interface portion 104.

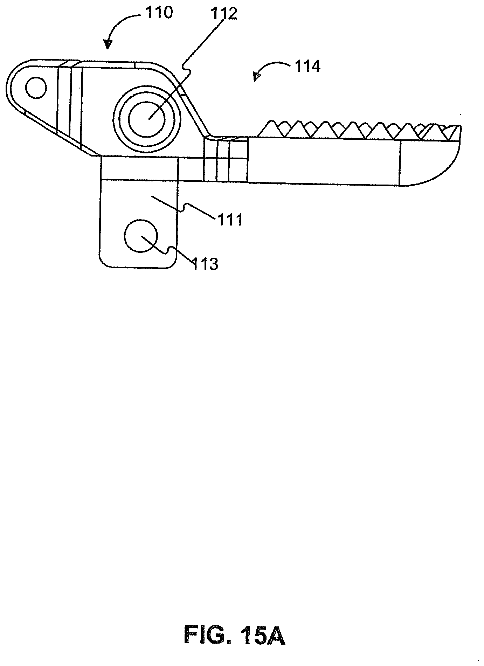

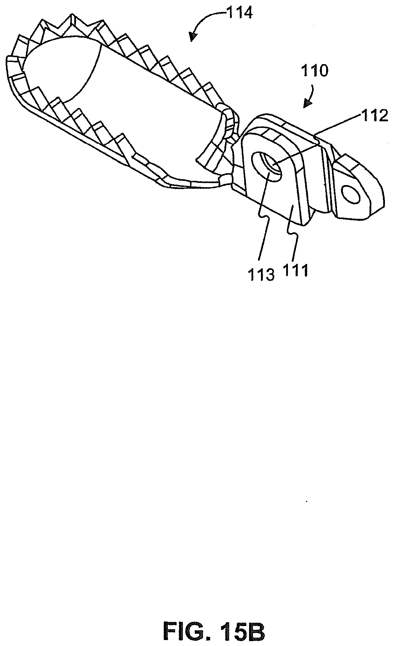

In various examples, the bends 103 need not make the end portion of the wire 101 into necessarily a roughly Z-shaped configuration. For example, the bends 103 could form the end portion of the wire 101 into a roughly U-shaped configuration. In addition, the bends 103 may be formed using any method known in the art. Furthermore, the dimples 201 may be formed using any method known in the art. For example, material may be soldered on and/or attached to the tang portion 200 using an adhesive to form dimples 201. Additionally, the thickness of the tang portion need not be increased by placing a dimple, as a portion of the tang portion may be folded over to increase the thickness. For example, in a tang portion manufactured from material having a thickness of about 0.0178 cm (0.007 inches), folding over the material would create a tang portion with a thickness of about 0.0356 cm (0.014 inches). Figs. 15A and 15B described below illustrate this concept as it relates to the axle hole of the jaw. The dimple 201 and/or tang portion 200 may be of any desired shape, size, dimensions, and/or configurations. For example, all the dimensions listed above are exemplary only.

In an example, a tang portion of an end effector assembly of a medical device may have a widened and/or thickened portion, for example, by folding over material in a portion of the tang around the axle hole. As shown in Figs. 15A-15B, a tang portion 110 of an end effector, such as a jaw 114, may be formed such that it has an additional portion 111 extending from the tang portion 110. The additional portion 111 has through hole 113 with substantially the same diameter as an axle hole 112 of the rest of the tang portion 110. The additional portion 111 may then be folded over such that the through hole 113 is aligned with the axle hole 112. For example, a tang portion 110 may be stamped from a material having a thickness of about 0.0178 cm (0.007 inches). Thus, both the tang portion 110 and the additional portion 111 have a width of about 0.0178 cm (0.007 inches). When folded over, the combined width of the tang portion 110 and the additional portion 111 becomes about 0.0356 cm (0.014 inches). A wider tang portion 110, and particularly a longer axle hole (the combined holes 112 and 113), may be advantageous because it imparts a wider footprint to the jaw mechanism, which may increase the stability and/or precision of the jaw, for example, during the clamping of opposing jaws.

In various examples, the tang portion may be widened by forming and then folding over multiple additional portions, for example, three additional portions. The width and/or thickness of other portions of a medical device, including other portions of the end effectors and/or end effector assembly, may be increased using this method. The folded over portion and/or tang portion may be of any desired shape, size, dimensions, and/or configurations. For example, all the dimensions listed above are exemplary only.

In another example, a tang portion of an end effector assembly of a medical device may have a portion configured to substantially prevent contact between an edge of the end effector and, for example, a tube-like member (such as an endoscope channel) in which the end effector assembly is configured to extend through or other external object. For example, in endoscopic applications, the jaws of a biopsy forceps device will follow the curvature of the endoscope. As the jaws pivot within an endoscope channel, the proximal tang behind the pivot may contact the channel wall. Biopsy jaws, including stamped biopsy jaws, may include sharp edges that may damage the endoscope channel.

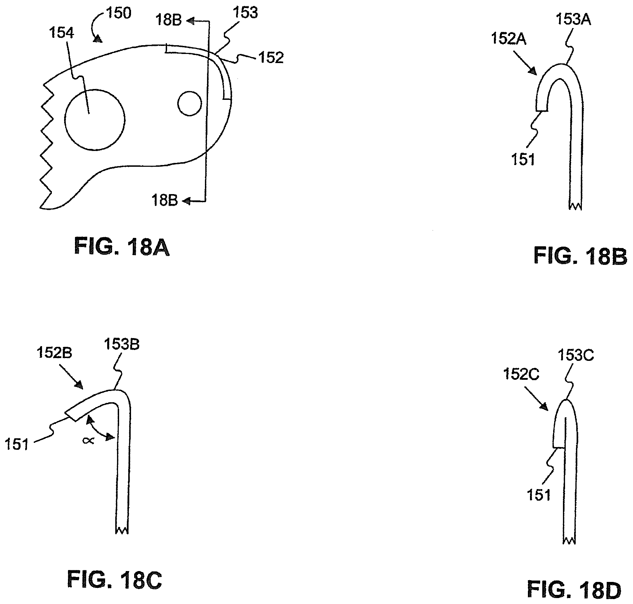

In an example, as shown in Figs. 18A-18D, a portion 152 of the tang 150 may be folded over so as to substantially prevent an edge 151 of the tang 150 from contacting the inside of an endoscope channel. Instead, a smooth folded portion of the tang having a greater area will contact the endoscope channel. The portion 152 may be disposed on a proximal portion of the tang, however, the portion may also be disposed on any other suitable portion of the end effector. As shown in Fig. 18B, the portion 152A may be curved, however, the portion 152B may also be more sharply folded over as shown in Fig. 18C, or substantially completely folded over as shown in Fig. 18D (i.e., a portion of the folded over portion 152C substantially contacts another portion of the tang 150) so that the portion 152C may be substantially parallel with the tang 150.

The tang 150 may have an outer periphery 153 along its entire circumference. At an apex between the portion 152 and the rest of the tang 150, the outer periphery 153 may be the portion of the tang 150 that comes into contact with the inside of the endoscope channel, for example, as the end effector pivots about a pivot hole 154 of the tang 150. The outer periphery 153A, 153B, 153C at that apex is shown on the respective Figs. 18B-18D, and preferably has a smooth surface.

In various examples, the folded over portion may be folded on any side of the tang and/or may have any desired geometric configuration. For example, the folded over portion may form any desired angle α (see Fig. 18C) with the tang, e.g., more than 90 degrees, less than 90 degrees, and/or substantially 0 degrees. Manufacturing a folded over portion with an angle of more than 90 degrees relative to the remainder of the tang may be easier than manufacturing a folded over portion an angle of less than 90 degrees. However, a folded over portion with a less than a 90 degree angle to the tang may be more effective in substantially preventing contact between a sharp edge of the end effector and the endoscope channel. In examples, the folded over portion may have a substantially rounded shape (e.g., having a constant radius or a variable radius), for example, to present a smooth, non-damaging contact between the tang and the endoscope channel. The folded over portion may have a semi-circular shape of more than 180 degrees, less than 180 degrees, or substantially equal to 180 degrees. In a further example, the tang may have multiple portions configured to substantially prevent contact between an edge of the end effector and the endoscope channel.

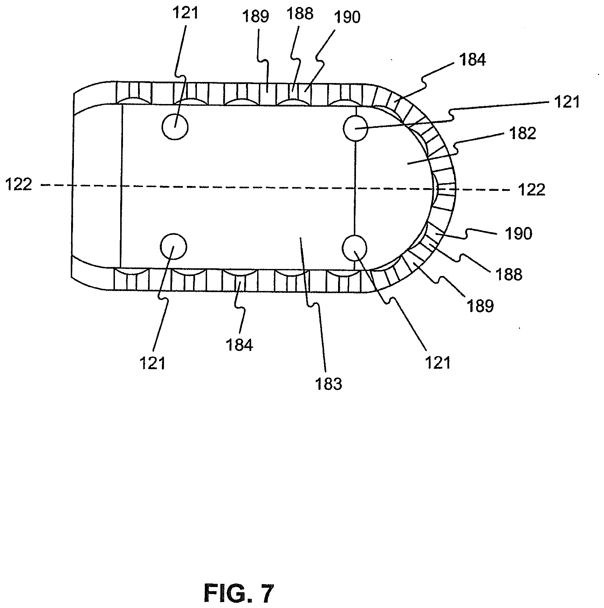

Examples include a medical device with holes in various portions of the medical device, including through the end effectors. For example, as shown in Fig. 7, a jaw 82 of a jaw assembly may have fenestration holes 121 in different portions of jaw 82. Fenestration holes 121 may assist in removing biopsy samples from the jaw 82, for example, by allowing fluid to enter the jaw 82 through the fenestration holes 121, flow between the biopsy sample and the jaw 82, and thus allow the biopsy sample to be flushed out of the jaw 82. The fenestration holes 121 may be disposed off a centerline 122 of the jaw 82. This may be advantageous as when the jaw 82 is placed down a channel, for example the working channel of an endoscope, because the jaw 82 may contact the inner wall of the channel substantially along its centerline 122, the channel will not come into contact with the fenestration holes 121. This may be desirable, for example, because contact between the holes 121 and the channel may cause the holes 121 to catch portions of the channel. This may cause damage to the channel and/or prevent the movement of the medical device with respect to the channel.

In various examples, the holes 121 may have any shape, for example, round, circular, oblong, square, and triangular. The holes 121 may also have of any size and have any desired dimensions. There may be any number of holes 121 on any portion of the medical device, but what is disclosed here are holes 121 that are not substantially located on the centerline 122 of the medical device and/or portions of the medical device that may come into contact with a channel and/or another object external to the medical device. The holes 121 need not necessarily be on portions of the medical device that completely preclude the holes 121 from coming into contact with the channel and/or another object external to the medical device, but may be on a portion where such contact is reduced or minimal relative to other portions of the medical device.

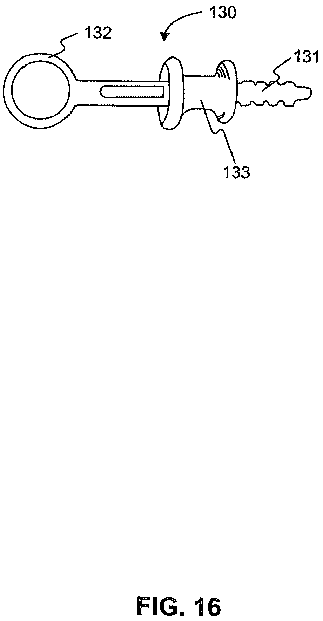

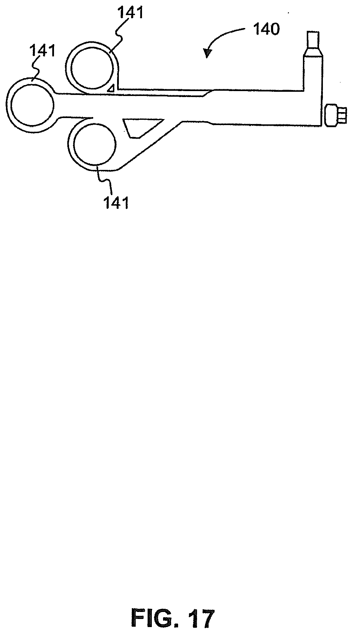

Examples include a medical device with user-interface portions configured to reduce stress (i.e. force) on the operator. For example, the handle of a medical device (e.g., an endoscopic instrument with a handle portion) may have soft-grip features. The entire handle may comprise the soft-grip features, or portions of the handle may have soft grip features, for example, those portions that accommodate a user's fingers. For example, in a handle 130 comprising a ring portion 132, an elongate portion 131, and a spool portion 133 disposed around the elongate portion 131, as shown in Fig. 16, the soft-grip features may be incorporated into the ring portion 132 and/or the spool portion 133. In another example, in a handle 140 comprising three-rings 141, as shown in Fig. 17, the soft-grip features may be incorporated into one or more of the three rings 141. The soft-grip feature may be a low durometer material, for example, santoprene or urethane, incorporated into the medical device. The soft-grip features reduce stress on the operator, for example, by reducing the stress on their hands, and have a more comfortable ergonomic feel. The reduction in stress on the user may allow the user to perform more procedures than with a medical device without the soft-grip features.

In various examples, any soft material may be used as soft-grip features, for example, rubber and/or rubbery thermoplastics. The soft-grip features may be placed on any portion of the medical device, for example, that have the potential to be handled by a user, provided that it does not otherwise interfere with the operation of the medical device. The soft-grip features may also be varied across portions of the device. For example, portions of the device may have different materials with different durometers.

Examples include a medical device having portions with variable stiffness. For example, in endoscopic instruments with an elongate member, portions of the elongate member may have variable stiffness. Some portions of the elongate member may be more flexible, for example, to allow the elongate member to be navigated through areas of the body having curves (i.e., tubular regions with greater tortuosity). Because of the flexibility, at least these portions of the elongate member may easily bend around even sharp curves, for example, in the gastrointestinal tract. Other portions of the elongate member may be more rigid, for example, to allow the elongate member to be properly advanced through areas of the body (e.g., tubular regions). Because of the rigidity, at least these portions of the elongate member can be pushed through, for example, the gastrointestinal tract.

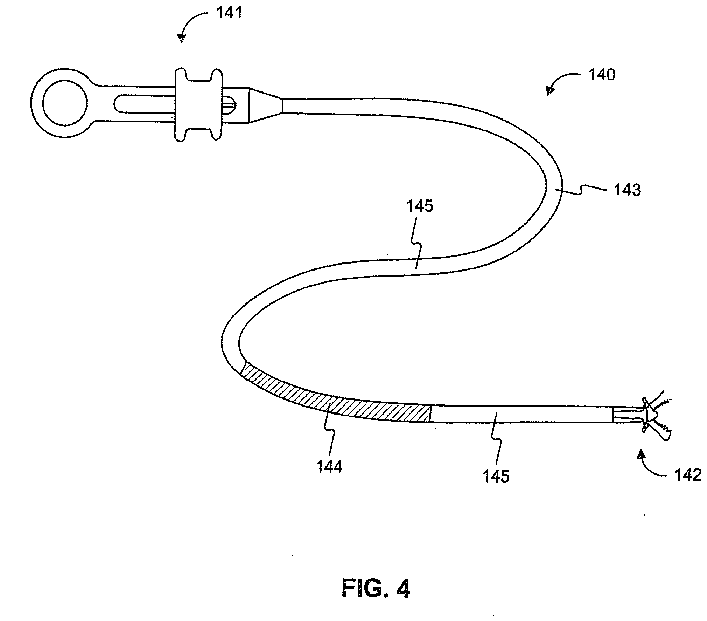

In an example, Fig. 4 shows an endoscopic instrument 140 with a handle 141 and an end effector assembly 142 connected by an elongate member 143. The elongate member 143 may have a diameter of about 2.4 mm and a length of about 350 cm. However, any other dimensions suitable for its intended use are also possible. The entire elongate member 143 has a constant strength and feel from its proximal end to distal end, however, a portion 144 of the distal third of the elongate member 143 proximal to the distal end effector assembly has a lower stiffness than the other portions 145 of the elongate member 143. Methods of reducing the stiffness of the desired portion 144 of the elongate member 143 include reducing the diameter of the elongate member 143 in the targeted area, and/or varying the material used in the elongate member 143 such that the lower stiffness portion 144 is comprised of a more flexible material than the higher stiffness portions 145.

In various examples, the elongate member may have its rigidity varied along any portion of the elongate member, may have multiple portions with multiple levels of stiffness, and/or may be manufactured using any method known in the art.

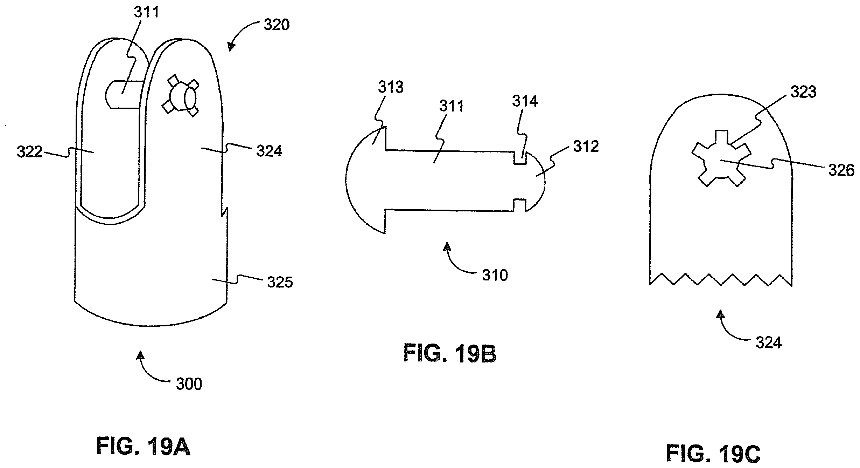

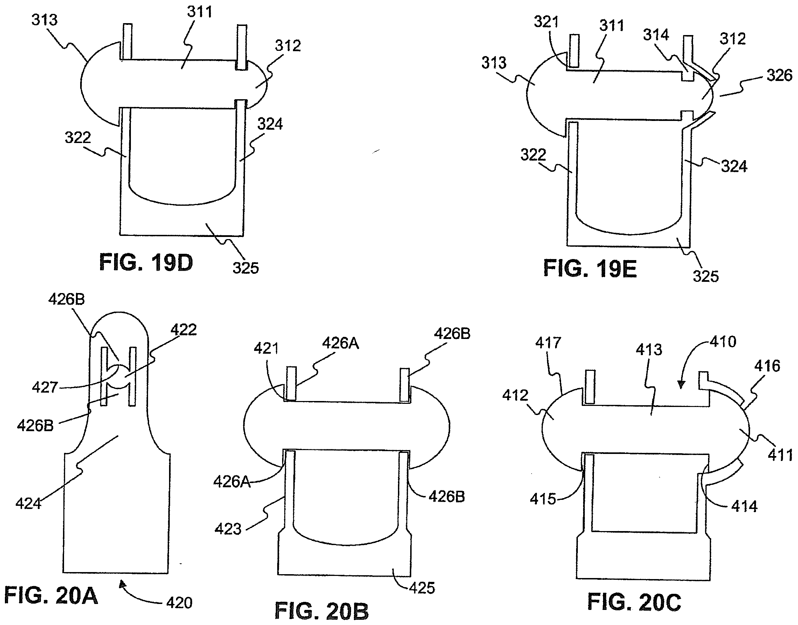

Examples include a clevis assembly. An exemplary embodiment of a clevis assembly 300 is shown in Figs. 19A-19E. The clevis assembly 300 may include an axle 310 and a clevis 320.

The axle 310 may be generally elongate in shape and configured to be used with clevis 320. The axle 310 may have a central portion 311 disposed between ends 312, 313. The central portion 311 may be substantially cylindrical in shape and may be configured to be placed through a hole 321 on one of the arms 322 of the clevis 320. The central portion 311 may also be configured to accommodate a portion of an end effector assembly, such as the proximal tang portions of biopsy jaws.

One end 313 of the axle 310 may be configured to prevent the end 313 from being placed through the hole 321 on the clevis arm 322. The end 313 may include an enlarged head with a shoulder. The head may be substantially hemispherical in shape, however, the end 313 may also have any suitable shape or configuration to prevent its extension through the hole 321.

The end 312 may be substantially round in shape, and may have a groove 314 that separates the end 312 from the central portion 311. The groove 314 may extend all the way around the axle 310, and may be configured to receive a portion of one or more of the protrusions, or cantilever arms, 323 extending around a hole 326 defined by another clevis arm 324.

The clevis 320 may have a base 325 from which arms 322, 324 extend. The arms 322, 324 may be substantially similar in shape, however, they may also have different shapes or configurations. One arm 322 has hole 321 configured to accommodate a portion of axle 310, for example, the central portion 311 of axle 310. Another arm 324 has a hole 326 with a non-uniform edge 327 that is defined by one or more protrusions 323. The protrusions 323 may each have substantially the same shape, or may have different shapes and/or configurations (e.g., spacing). The holes 321, 326 may be substantially coaxial. The portion of the arm 324 defining the hole 326 may be configured to bend or deflect as axle 310 is placed through the hole 326. For example, as shown in Fig. 19E, the protrusions 323 may deflect away from the arm 322 as end 312 of the axle 310 is placed through the hole 326. The portion of the arm 324 defining the hole 326 may also be configured to substantially return to its original configuration. For example, once the end 312 of the axle 310 has been placed through the hole 326 a suitable amount, the protrusions 323 may deflect or spring back toward the arm 322 and at least a portion of the protrusions 323 may become lodged in groove 314.

The portion of the arm 324 not defining the hole 326 and/or protrusions 323 may be configured to be rigid enough such that the arm 324 does not substantially bend or deflect while the protrusions 323 bend or deflect as the end 312 of the axle 310 is placed through the hole 326. For example, the portion of the arm 324 not defining the hole 326 may be thicker than the protrusions 323. The base 325 may also be configured to be more rigid than the arms 322, 324, for example, so as to not substantially bend or deflect while the protrusions 323 may bend or deflect as the end 312 of the axle 310 is placed through the hole 326. In another example, the portion of the arm 324 not defining the hole 326 and/or protrusions 323 may not have any particular configuration or rigidity such that the arm 324 does not substantially bend or deflect while the protrusions 323 bend or deflect as the end 312 of the axle 310 is placed through the hole 326. For example, arm 324 may simply have roughly the same thickness, rigidity, and/or metallic properties as the rest of the clevis assembly 300. In such cases, tooling may be used to prevent deflection of the arm 324. For example, the arm 324 may be placed between grippers, vices, or any other suitable tooling known in the art so as to substantially prevent deflection of the arm 324 in a direction substantially perpendicular to the surface of the arm 324 and/or substantially parallel to the longitudinal axis of the axle 311 (e.g., when the end 312 of the axle 310 is placed through the hole 326 and exerts force on the protrusions 323).

Another example of a clevis assembly 400 is shown in Figs. 20A-20C. The clevis assembly 400 may include an axle 410 and a clevis 420.

The axle 410 may have two ends 411, 412 disposed around a central portion 413. The central portion 413 may be substantially cylindrical in shape and may be configured to be disposed in holes 421, 422 on arms 423, 424 on the clevis 420. The central portion 413 may also be configured to accommodate a portion of an end effector assembly, such as the proximal tang portions of biopsy jaws.

The ends 411, 412 may have a generally rounded shape and may be configured to prevent the ends 411, 412 from being placed through at least one of the holes 421. For example, the ends 411, 412 may include an enlarged head and a shoulder. The head may be substantially hemispherical in shape, however, the ends 411, 412 may have any suitable shape or configuration. An inner surface 414, 415 of the ends 411, 412 may be configured to prevent the rest of the end 411, 412 from being placed through holes 421, 422. An outer surface 416, 417, however, may be configured to be placed through at least one of the holes 421, 422. The ends 411, 412 may have substantially the same shape and configuration, or may have different shapes and/or configurations. For example, one of the ends 411, 412 may be configured so that it may not be placed through at least one of the holes 421, 422.

The clevis 420 may have a base 425 from which arms 423, 424 extend. The arms 423, 424 may have substantially similar shapes, or may have different shapes and/or configurations. One or more of the arms 423, 424 may define a hole 421, 422 with one or more protrusions 426A, 426B adjacent portions of the hole 421, 422. The protrusions 426A, 426B may have the same shape, or may have different shapes. The protrusions 426A, 426B may define substantially rounded inner edges 427 that are configured, for example, to define portions of a circle. The protrusions 426A may be configured to deflect toward arm 424 as end 411 is placed through the hole 421. The protrusions 426B may be configured to deflect away from arm 423 as end 411 is placed through the hole 422. As shown in Fig. 20C, when an outer surface 416 of an end 411 of an axle 410 is pressed against the protrusions 426B, the protrusions 426B may deflect as the end 411 is advanced through hole 422. Once the end 411 has suitably advanced through the hole 422, the protrusions 426B may reversibly deflect toward the arm 423 such that the inner edges 427 are adjacent an outer surface of the central portion 413. In such a configuration, the inner surfaces 414, 415 of the ends 411, 412 may be adjacent outer surfaces of the arms 423, 424. The same may substantially be true for hole 421 and protrusions 426A, except that the outer surface 416 of the end 411 of the axle 410 may first come into contact with an outer surface of arm 423, and the protrusions 426A may deflect inward (i.e., toward arm 424).

The portion of the arm 423, 424 not defining the hole 421, 422 and/or protrusions 426A, 426B may be configured to be rigid enough such that the arm 423, 424 does not substantially bend or deflect while the protrusions 426A, 426B may bend or deflect as the end 411 of the axle 410 is placed through the hole 421,422. For example, the portion of the arm 423, 424 not defining the hole 421, 422 may be thicker than the protrusions 426A, 426B. The base 425 may also be configured to be more rigid than the arms 423, 424, for example, so as to not substantially bend or deflect while protrusions 426A, 426B may bend or deflect as the end 411 of the axle 410 is placed through the hole 421, 422. In another example, the portion of the arm 423, 424 not defining the hole 421, 422 and/or protrusions 426A, 426B may not have any particular configuration or rigidity such that the arm 423, 424 does not substantially bend or deflect while the protrusions 426A, 426B bend or deflect as the end 411 of the axle 410 is placed through the hole 426A, 426B. For example, arms 423, 424 may have roughly the same thickness, rigidity, and/or metallic properties as the rest of the clevis assembly 420. In such a case, tooling may be used to prevent deflection of the arm 423, 424. For example, the arm 423, 424 may be placed between grippers, vices, or any other suitable tooling known in the art so as to substantially prevent deflection of the arm 423, 424 in a direction substantially perpendicular to the surface of the arm 423 424 and/or substantially parallel to the longitudinal axis of the axle 410 (e.g., when the end 411 of the axle 410 is placed through the hole 421, 422 and exerts force on the protrusions 426A, 426B).

In various examples, each arm of the clevis may define a hole with protrusions configured to deflect and then return to its original configuration as an axle is placed therethrough, substantially as set forth above. However, in other examples, clevis arms may have different configurations. For example, one of the arms may define a hole with protrusions configured to deflect and then return to its original configuration as an axle is placed therethrough, however, the other arm may define a hole without protrusions that is otherwise configured to allow an end of an axle to pass through the hole without substantially deflecting any portion of the arm. In such a configuration, one end of the axle may have a small enough size and/or shape to pass through the hole on one of the arms and then deflect the protrusions adjacent the hole on the other arm as the end passes therethrough.

There may be several advantages to having a clevis assembly with an axle and clevis configuration according to one of the examples set forth herein, for example, Figs. 19A-19E and 20A-20C. One advantage is the elimination of a rivet and the use of expensive riveting equipment to manufacture the clevis assembly. Another advantage is that the clevis assembly may be assembled quickly and through an automated process. A further advantage is that the axle may be solid and thus less expensive than hollow axles which may be used in other clevis assembly configurations. Yet another advantage is that the axle may not include sharp points or edges that may damage the walls of a working channel of an endoscope through which the clevis assembly may be placed. Still another advantage is that the groove may be accurately and precisely placed on the axle such that when the clevis assembly is assembled and the protrusions on the hole of one of the arms are disposed in the groove, the resulting distance between the arms may be precisely controlled and/or ideally manufactured for the intended use of the clevis assembly.

In various examples, all aspects set forth herein may be used in conjunction with any medical device, instrument, or procedure, and/or any non-medical device, instrument, or procedure.

Other examples will be apparent to those skilled in the art from consideration of the specification and practice of the examples disclosed herein. It is intended that the specification and examples be considered as exemplary only, with the scope of the invention being defined by the following claims.

## Claims

1. A medical device (10) comprising:
a handle (11);
an end effector assembly (12); and
a member (13) connecting the handle to the end effector assembly,
wherein the end effector assembly comprises opposing jaw portions (183) each including a plurality of teeth (184),
wherein each of the plurality of teeth includes a crest (188), a root (189), a first intermediate portion, which is a diagonal portion (190) extending between a sharp, pointed tip of the crest and the root, and a second intermediate portion, which is a diagonal portion extending between a sharp, pointed tip of the crest and a root of an adjoining tooth,
wherein surfaces of first intermediate portions contact surfaces of second intermediate portions of opposing jaw portions when the opposing jaw portions are in a closed configuration,
**characterized in that**
the root of each tooth has a recessed portion configured to accommodate the tip of the crest of an opposing tooth, and
wherein a gap exists between the tip and the root of opposing teeth when the opposing jaw portions are in the closed configuration.

2. The medical device of claim 1, wherein at least one opposing jaw portion has a tissue-receiving portion configured to retain at least one tissue sample, the tissue-receiving portion defining at least one hole configured so as to prevent contact between an edge of the hole and a tube-like member in which the end effector assembly is configured to extend through.

3. The medical device of claim 2, wherein the tissue-receiving portion has at least one roughened surface.

4. The medial device claim 3, wherein the roughened surface is formed by one of grit blasting, media tumbling, plating, sputtering, photo-etching, acid-etching, and plasma coating.

5. The medical device of any of the preceding claims, wherein the handle is a soft-grip handle.

6. The medical device of any of the preceding claims, wherein the at least one root is a partial, circular cutout.

7. The medical device of claim 6, wherein a center of the cutout is displaced vertically relative to adjacent intermediate portions.

8. The medical device of claim 6, wherein a center of the shape is displaced horizontally relative to a center of adjacent intermediate portions.

9. The medical device of any of the preceding claims, wherein the at least one root is a U-shaped groove.

10. The medical device of claim 9, wherein a center of the U-shaped groove is displaced vertically relative to adjacent intermediate portions.

11. The medical device of claim 9, wherein a center of the U-shaped groove is displaced horizontally relative to a center of adjacent intermediate portions.

12. The medical device of any of the preceding claims, wherein each opposing jaw portion includes a tang defining a mounting hole configured to receive one of a wire and an axle,
wherein the tang includes a portion disposed around the mounting hole that has a thickness greater than a thickness of other portions of the tang, and
wherein a section of the tang defining a through-hole is folded so that the through-hole is aligned with the mounting hole.

13. The medical device of any of the preceding claims, wherein edges of each opposing jaw portion, other than a cutting edge of each opposing jaw portion, are non-sharp.

14. The medical device of any of the preceding claims, wherein the tips of the crests of the plurality of teeth are sharp.

## Patentansprüche

1. Medizinische Vorrichtung (10) mit:
einem Griff (11);
einer Endeffektoranordnung (12); und
einem Element (13), das den Griff (11) mit der Endeffektoranordnung (12) verbindet,
wobei die Endeffektoranordnung ein Paar aus gegenüberliegenden Backenabschnitten (183) mit jeweils mehreren Zähnen (184) aufweist,
wobei jeder der mehreren Zähne einen Kamm (188), eine Wurzel (189), einen ersten Zwischenabschnitt, der ein sich zwischen einer scharfen, zugespitzten Spitze des Kammes und der Wurzel erstreckender Diagonalabschnitt (190) ist, und einen zweiten Zwischenabschnitt aufweist, der ein sich zwischen einer scharfen, zugespitzten Spitze des Kammes und einer Wurzel eines angrenzendes Zahnes erstreckender Diagonalabschnitt ist,
wobei Oberflächen von ersten Zwischenabschnitten mit Oberflächen von zweiten Zwischenabschnitten gegenüberliegender Backenabschnitte in Kontakt kommen, wenn sich die gegenüberliegenden Backenabschnitte in einer geschlossenen Konfiguration befinden,
**dadurch gekennzeichnet, dass**
die Wurzel eines jeden Zahnes einen vertieften Abschnitt aufweist, der konfiguriert ist, die Spitze des Kammes eines gegenüberliegenden Zahnes aufzunehmen, und
wobei zwischen der Spitze und der Wurzel gegenüberliegender Zähne eine Lücke besteht, wenn sich die gegenüberliegenden Backenabschnitte in der geschlossenen Konfiguration befinden.

2. Medizinische Vorrichtung nach Anspruch 1, wobei mindestens ein gegenüberliegender Backenabschnitt einen Gewebe-Aufnahmeabschnitt aufweist, der konfiguriert ist, mindestens eine Gewebeprobe zu halten, wobei der Gewebe-Aufnahmeabschnitt mindestens ein Loch definiert, das konfiguriert ist, Kontakt zwischen einer Kante des Lochs und einem rohrartigen Element zu verhindern, durch welches sich hindurch zu erstrecken die Endeffektoranordnung (12) konfiguriert ist.

3. Medizinische Vorrichtung nach Anspruch 2, wobei der Gewebe-Aufnahmeabschnitt mindestens eine aufgeraute Oberfläche aufweist.

4. Medizinische Vorrichtung nach Anspruch 3, wobei die aufgeraute Oberfläche durch eines aus Sandstrahlen, Medienscheuern, Galvanisieren, Sputtern, Photoätzen, Säureätzen und Plasmabeschichten erzeugt wird.

5. Medizinische Vorrichtung nach einem der vorhergehenden Ansprüche, wobei der Griff ein weicher Griff ist.

6. Medizinische Vorrichtung nach einem der vorhergehenden Ansprüche, wobei die mindestens eine Wurzel eine runde Teilaussparung ist.

7. Medizinische Vorrichtung nach Anspruch 6, wobei ein Mittelpunkt der Aussparung relativ zu benachbarten Zwischenabschnitten vertikal verschoben ist.

8. Medizinische Vorrichtung nach Anspruch 6, wobei ein Mittelpunkt der Form relativ zu einem Mittelpunkt benachbarter Zwischenabschnitte horizontal verschoben ist.

9. Medizinische Vorrichtung nach einem der vorhergehenden Ansprüche, wobei die mindestens eine Wurzel eine U-förmige Nut ist.

10. Medizinische Vorrichtung nach Anspruch 9, wobei ein Mittelpunkt der U-förmigen Nut relativ zu benachbarten Zwischenabschnitten vertikal verschoben ist.

11. Medizinische Vorrichtung nach Anspruch 9, wobei ein Mittelpunkt der U-förmigen Nut relativ zu benachbarten Zwischenabschnitten horizontal verschoben ist.

12. Medizinische Vorrichtung nach einem der vorhergehenden Ansprüche, wobei jeder gegenüberliegende Backenabschnitt eine Greifangel beinhaltet, die ein Montageloch definiert, das konfiguriert ist, entweder einen Draht oder eine Achse aufzunehmen, und
wobei die Greifangel einen um das Montageloch herum angeordneten Abschnitt hat, welcher eine Dicke größer als die Dicke von anderen Abschnitten der Greifangel hat, und
wobei ein Abschnitt der Greifangel, die ein Durchgangsloch definiert, gefaltet ist, sodass das Durchgangsloch an dem Montageloch ausgerichtet ist.

13. Medizinische Vorrichtung nach einem der vorhergehenden Ansprüche, wobei Kanten jedes gegenüberliegenden Backenabschnitts, mit Ausnahme von einer Schneidkante eines jeden gegenüberliegenden Backenabschnitts, nicht scharf sind.

14. Medizinische Vorrichtung nach einem der vorhergehenden Ansprüche, wobei die Spitzen der Kämme der mehreren Zähne scharf sind.

## Revendications

1. Dispositif médical (10) comprenant :
une manette (11) ;
un ensemble d'effecteur terminal (12) ; et
un élément (13) raccordant la manette à l'ensemble d'effecteur terminal,
dans lequel l'ensemble d'effecteur terminal comprend des parties de mâchoire (183) opposées comprenant chacune une pluralité de dents (184),
dans lequel chacune de la pluralité de dents comprend une crête (188), une base (189), une première partie intermédiaire qui est une partie diagonale (190) s'étendant entre une pointe pointue tranchante de la crête et la base, et une seconde partie intermédiaire qui est une partie diagonale s'étendant entre une pointe pointue tranchante de la crête et une base d'une dent attenante,
dans lequel les surfaces des premières parties intermédiaires sont en contact avec les surfaces des secondes parties intermédiaires des parties de mâchoire opposées lorsque les parties de mâchoire opposées sont dans une configuration fermée,
**caractérisé en ce que** :
la base de chaque dent a une partie évidée configurée pour loger la pointe de la crête de la dent opposée, et
dans lequel il existe un espace entre la pointe et la base des dents opposées lorsque les parties de mâchoire opposées sont dans la configuration fermée.

2. Dispositif médical selon la revendication 1, dans lequel au moins une partie de mâchoire opposée a une partie de réception de tissu configurée pour retenir au moins un échantillon de tissu, la partie de réception de tissu définissant au moins un trou configuré pour empêcher le contact entre un bord du trou et un élément en forme de tube dans lequel l'ensemble d'effecteur terminal est configuré pour s'étendre à travers ce dernier.

3. Dispositif médical selon la revendication 2, dans lequel la partie de réception de tissu a au moins une surface rugueuse.

4. Dispositif médical selon la revendication 3, dans lequel la surface rugueuse est formée par une méthode parmi le décapage au sable, le polissage par billes, l'électrodéposition, la pulvérisation cathodique, la photogravure, la gravure aux acides et le plaquage au plasma.

5. Dispositif médical selon l'une quelconque des revendications précédentes, dans lequel la manette est une manette à prise souple.

6. Dispositif médical selon l'une quelconque des revendications précédentes, dans lequel la au moins une base est une découpe circulaire partielle.

7. Dispositif médical selon la revendication 6, dans lequel un centre de la découpe est verticalement déplacé par rapport aux parties intermédiaires adjacentes.

8. Dispositif médical selon la revendication 6, dans lequel un centre de la forme est horizontalement déplacé par rapport à un centre des parties intermédiaires adjacentes.

9. Dispositif médical selon l'une quelconque des revendications précédentes, dans lequel la au moins une base est une rainure en forme de U.

10. Dispositif médical selon la revendication 9, dans lequel un centre de la rainure en forme de U est déplacé verticalement par rapport aux parties intermédiaires adjacentes.

11. Dispositif médical selon la revendication 9, dans lequel un centre de la rainure en forme de U est déplacé horizontalement par rapport à un centre des parties intermédiaires adjacentes.

12. Dispositif médical selon l'une quelconque des revendications précédentes, dans lequel chaque partie de mâchoire opposée comprend une queue définissant un trou de montage configuré pour recevoir l'un parmi un fil et un essieu,
dans lequel la queue comprend une partie disposée autour du trou de montage qui a une épaisseur supérieure à une épaisseur des autres parties de la queue, et
dans lequel une section de la queue définissant un trou débouchant est pliée de sorte que le trou débouchant est aligné avec le trou de montage.

13. Dispositif médical selon l'une quelconque des revendications précédentes, dans lequel les bords de chaque partie de mâchoire opposée, autres qu'un bord de coupe de chaque partie de mâchoire opposée, sont non tranchants.

14. Dispositif médical selon l'une quelconque des revendications précédentes, dans lequel les pointes des crêtes de la pluralité de dents sont tranchantes.
